# EUROPEAN PATENT APPLICATION

(11) **EP 4 046 637 A1**
(43) Date of publication of application: **24.08.2022**
(21) Application number: 20876076.9
(22) Date of filing: 07.10.2020
(51) Int. Cl.: A61K 31/436, A61P 25/00, A61P 25/08, A61K 9/06

(54) **EXTERNAL PREPARATION FOR TREATING EPILEPSY OR AUTISM SPECTRUM DISORDER**

(30) Priority: 16.10.2019 JP 2019189091
(71) Applicant: OSAKA UNIVERSITY, Suita-shi Osaka 565-0871 (JP)
(72) Inventor: KANEDA Mari, Suita-shi, Osaka 565-0871 (JP)
(74) Representative: Krauss, Jan
(86) International application number: PCT/JP2020/037986
(87) International publication number: WO 2021/075328

(57) **Abstract**

It is an object to provide a highly safe and easy-to-administer pharmaceutical preparation for treating epilepsy, pharmaceutical preparation for treating an autism spectrum disorder, and/or pharmaceutical preparation for treating an anxiety disorder. [Solving Means] A topical preparation containing as an active ingredient at least one selected from the group consisting of sirolimus and a sirolimus derivative is a pharmaceutical preparation that is easy to administer, and is highly safe as a therapeutic agent for epilepsy, an autism spectrum disorder, and/or an anxiety disorder. Suitable examples of the active ingredient include sirolimus, everolimus, temsirolimus, ridaforolimus, and zotarolimus.

## Description

### Technical Field

The present invention relates to treatment of epilepsy, treatment of an autism spectrum disorder, and treatment of an anxiety disorder.

### Background Art

The inventor of the present invention and others found that skin lesions with tuberous sclerosis complex were treatable by using a topical preparation containing sirolimus (rapamycin), and developed the topical preparation as a pharmaceutical product (Patent Literature 1).

Epilepsy is a chronic brain disease in which consciousness disorder, convulsions, and the like are caused in a seizure-like manner by excessive electrical excitation of neurons in the brain. There are reports that oral administration of sirolimus or everolimus may be effective against epilepsy (Non Patent Literatures 1 to 3). However, an antiepileptic drug needs to be continuously administered, and hence a safer pharmaceutical preparation is required. In addition, the pharmaceutical preparation is also required to be easy to administer in order to keep satisfactory adherence and continue its administration.

Autism spectrum disorder refers to nearly the same concept as "pervasive developmental disorder", and includes autism, Asperger syndrome, and the like. An experiment of intraperitoneal administration on mice has suggested that sirolimus may achieve recovery from autism (Non Patent Literature 4). In addition, the inventor of the present invention and others have reported that oral intake of everolimus ameliorates autism (Non Patent Literature 5). A drug for an autism spectrum disorder also needs to be continuously administered, and hence a safer pharmaceutical preparation is required. In addition, the pharmaceutical preparation is also required to be easy to administer in order to keep satisfactory adherence and continue its administration.

### Citation List

### Patent Literature

[PTL 1] WO 2012/105521 A1

### Non Patent Literature

[NPL 1] French JA, et al., Lancet 2016 Oct 29; 388(10056): 2153-2163
[NPL 2] Krueger DA, et al., Ann Neurol 2013; 74: 679-87
[NPL 3] Cardamone M, et al., J Pediatr 2014; 164: 1195-200
[NPL 4] Sato A, et al., Nature Communications 2012; 3: 1292
[NPL 5] Ishii R, et al., Neuropsychiatric Electrophysiology 2015 1: 6

### Summary of Invention

### Technical Problem

An object of the present invention is to provide a highly safe and easy-to-administer topical preparation for treating epilepsy, topical preparation for treating an autism spectrum disorder, and/or topical preparation for treating an anxiety disorder.

### Solution to Problem

The inventor of the present invention has found that epilepsy, an autism spectrum disorder, and/or an anxiety disorder can be treated by topically applying sirolimus or a sirolimus derivative. Thus, the inventor has completed the present invention. That is, the present invention encompasses the following aspects.
1. A topical preparation for treating epilepsy, including as an active ingredient at least one selected from the group consisting of sirolimus and a sirolimus derivative.
2. The topical preparation according to the above-mentioned item 1, wherein the sirolimus derivative is at least one selected from the group consisting of everolimus, temsirolimus, ridaforolimus and zotarolimus.
3. The topical preparation according to the above-mentioned item 1 or 2, wherein the epilepsy is epilepsy accompanying tuberous sclerosis complex.
4. The topical preparation according to any one of the above-mentioned items 1 to 3, wherein the topical preparation is for use by administration to a head.
5. The topical preparation according to any one of the above-mentioned items 1 to 4, further including a lower alcohol.
6. A topical preparation for treating an autism spectrum disorder, including as an active ingredient at least one selected from the group consisting of sirolimus and a sirolimus derivative.
7. The topical preparation according to the above-mentioned item 6, wherein the autism spectrum disorder is autism.
8. The topical preparation according to the above-mentioned item 6, wherein the autism spectrum disorder is a disorder in building an interpersonal relationship in an autism spectrum disorder, and/or perseveration and obsession in an autism spectrum disorder.
9. The topical preparation according to the above-mentioned item 6, wherein the autism spectrum disorder is an autism spectrum disorder accompanying tuberous sclerosis complex.
10. A topical preparation for treating an anxiety disorder, including as an active ingredient at least one selected from the group consisting of sirolimus and a sirolimus derivative.
11. The topical preparation according to the above-mentioned item 10, wherein the anxiety disorder is generalized anxiety disorder.
12. The topical preparation according to the above-mentioned item 10, wherein the anxiety disorder is an anxiety disorder accompanying tuberous sclerosis complex.
13. A method of treating epilepsy, comprising topically applying at least one selected from the group consisting of sirolimus and a sirolimus derivative.
14. The method according to the above-mentioned item 13, wherein the topically applying is performed to a head.
15. A method of treating an autism spectrum disorder, comprising topically applying at least one selected from the group consisting of sirolimus and a sirolimus derivative.
16. A method of treating an anxiety disorder, comprising topically applying at least one selected from the group consisting of sirolimus and a sirolimus derivative.

### Advantageous Effects of Invention

The topical preparation of the present invention exhibits an effect of treating the symptoms of epilepsy through percutaneous or transmucosal administration. The topical preparation of the present invention can suppress epileptic seizures and reduce the number of seizures.

The topical preparation of the present invention exhibits an effect of treating an autism spectrum disorder through percutaneous or transmucosal administration.

The topical preparation of the present invention exhibits an effect of treating an anxiety disorder through percutaneous or transmucosal administration.

The topical preparation of the present invention is a safe pharmaceutical preparation that exhibits its effect without increasing the blood concentration, and hence little affects other viscera and organs, with the result that side effects are alleviated. In addition, a method involving application or affixation to a skin or a mucous membrane is a simple administration method, and hence the topical preparation is a formulation suited for long-term continuous administration. In addition, the topical preparation can be administered to a patient or a child for whom oral administration is difficult. The topical preparation can be administered even when a symptom that makes oral administration difficult appears, for example, during an epileptic seizure.

### Brief Description of Drawings

FIG. 1 includes photographs of mice each having a sirolimus gel or a placebo applied to its shaved forehead.
FIG. 2 is a graph showing results obtained by applying a 0.2 wt% sirolimus gel or a placebo (only a base) to model mice and control mice (normal mice) once a day at 5 mg each (10 µg in terms of sirolimus), and counting the number of epileptic seizures for 7 days (n=4 in each group).
Homo represents tuberous sclerosis complex model mice, and Ct represents control mice (normal mice). ^{∗∗} P<0.01, ^{∗∗∗} P<0.001
FIG. 3 is a graph showing results obtained by intraperitoneally administering sirolimus (3 mg/kg/day) or saline to model mice and control mice (normal mice) once a day for 7 days (n=6 in each group). Homo represents tuberous sclerosis complex model mice, and Ct represents control mice (normal mice). ^{∗} P<0.05, ^{∗∗} P<0.01

### Description of Embodiments

The present invention is not limited to embodiments described below, and various changes may be made thereto within the scope of claims. Embodiments and Examples obtained by combining as appropriate technical means disclosed in different embodiments and Examples are also included in the technical scope of the present invention.

A topical preparation for treating epilepsy, a topical preparation for treating an autism spectrum disorder, and a topical preparation for treating an anxiety disorder according to the present invention each contain as an active ingredient at least one selected from the group consisting of sirolimus and a sirolimus derivative.

The sirolimus derivative is not particularly limited, and examples thereof may include everolimus, temsirolimus, ridaforolimus, and zotarolimus. Those sirolimus derivatives are each known to have nearly the same basic skeleton as the basic skeleton of sirolimus, and to have physiological activity comparable to that of sirolimus. Accordingly, each of those sirolimus derivatives may be used, as well as sirolimus, as the active ingredient according to one embodiment of the present invention.

Sirolimus is already in use as topical therapeutic drugs for a skin lesion accompanying tuberous sclerosis complex and the like, and has been recognized for its safety and absorbability in clinical use. Accordingly, a more preferred active ingredient is sirolimus.

The topical preparation for treating epilepsy, the topical preparation for treating an autism spectrum disorder, and the topical preparation for treating an anxiety disorder according to the present invention may each be used for the treatment of various symptoms to be developed in a patient with the disease of interest.

Herein, the "treatment" includes, for example, the following concepts.
(1) To prevent the development of one or more symptoms associated with the disease, or to reduce the risk of development thereof.
(2) To prevent the recurrence of one or more symptoms associated with the disease, or to reduce the risk of recurrence thereof.
(3) To alleviate or ameliorate one or more symptoms associated with the disease, or to eliminate the symptoms.
(4) To suppress the exacerbation of one or more symptoms associated with the disease, or to suppress the progression thereof.
(5) To reduce the rate of progression of the exacerbation of one or more symptoms associated with the disease.

Epilepsy is a disease that repeatedly causes epileptic seizures involving, for example, a sudden loss of consciousness and unresponsiveness, and the symptoms of epileptic seizures are diverse depending on in which region of the brain the disturbance or excitement of an electrical signal occurs. The topical preparation for treating epilepsy of the present invention can suppress epileptic seizures. The epileptic seizures to be treated by the present invention are not limited, and include all epileptic seizures. Of those, an epileptic seizure in focal cortical dysplasia type II is preferred. In addition, the topical preparation exhibits a suitable effect on the suppression of epileptic seizures accompanying tuberous sclerosis complex.

The autism spectrum disorder in the present invention is nearly the same as pervasive developmental disorder. The autism spectrum disorder in the present invention includes, for example, autism, Asperger syndrome, and other pervasive developmental disorders. The autism spectrum disorder has three symptoms (features): an interpersonal relationship disorder, a communication disorder, and a bias in interest or behavior (e.g., perseveration and obsession). The topical preparation of the present invention is suitably for use in treatment of autism. In addition, the topical preparation of the present invention is suitably for use in treatment of an autism spectrum disorder accompanying tuberous sclerosis complex. The topical preparation of the present invention is more suitably for use in treatment of autism accompanying tuberous sclerosis complex. In addition, the topical preparation of the present invention may be suitably used for amelioration of a disorder in building an interpersonal relationship, a reduction in sociability, a communication disorder, a bias in interest or behavior, perseveration and obsession, repetitive behavior, and/or a sensory abnormality in an autism spectrum disorder. The topical preparation of the present invention is suitably for use in treatment of a disorder in building an interpersonal relationship in an autism spectrum disorder and/or for use in treatment of perseveration and obsession therein. The topical preparation of the present invention is more suitably for use in treatment of a disorder in building an interpersonal relationship accompanying tuberous sclerosis complex and/or for use in treatment of perseveration and obsession accompanying tuberous sclerosis complex.

The anxiety disorder in the present invention includes, for example, generalized anxiety disorder and an anxiety disorder caused by a general medical condition. The topical preparation of the present invention is suitably for use in treatment of generalized anxiety disorder or for use in treatment of an anxiety disorder accompanying a general medical condition. The topical preparation may be suitably used for treatment of an anxiety disorder accompanying tuberous sclerosis complex.

The topical preparation for treating epilepsy and the topical preparation for treating an autism spectrum disorder of the present invention are each preferably a topical preparation for administration to the head. The administration site of the topical preparation of the present invention is preferably the head. The head includes, for example, the face, the parietal region, the occipital region, the temporal region, the nasal cavity, and the oral cavity, and a suitable example of the administration site is the face. An administration method involving application or affixation to the face is suitable. The topical preparation of the present invention is suitably a topical preparation for facial administration.

The topical preparation of the present invention may be used for humans and non-human mammals. Examples of the non-human mammals include mammals excluding humans. Examples of the mammals excluding humans include: even-toed ungulates, such as cattle, wild boars, pigs, sheep, and goats; odd-toed ungulates, such as horses; rodents, such as mice, rats, hamsters, and squirrels; Lagomorpha, such as rabbits; and carnivorans, such as dogs, cats, and ferrets. In addition, such non-human animal may be suitably a domestic animal or a companion animal (pet animal).

The administration route of the topical preparation of the present invention is, for example, percutaneous or transmucosal. Accordingly, the topical preparation of the present invention may be a gel, an ointment, a cream, a patch, a collunarium, a suppository, a poultice, a liniment, a lotion, an inhalant, a spray, and the like. The gel allows its active ingredient to be easily absorbed by a skin tissue as compared to the ointment or the like, and hence may be said to be a more preferred dosage form.

The topical preparation of the present invention can effectively treat epilepsy, an autism spectrum disorder, and/or an anxiety disorder through topical application. The topical preparation is a safe formulation that exhibits its therapeutic effect while causing little increase in blood concentration, and hence little affects other viscera and organs, with the result that side effects are alleviated.

The content of the active ingredient in the topical preparation of the present invention is not particularly limited. The following concentration is suitable from the viewpoint of suppressing the moving of the active ingredient into blood, to thereby suppress systemic side effects and provide a therapeutic effect.

The lower limit of the concentration of the active ingredient may be 0.01 wt% or more, 0.02 wt% or more, 0.03 wt% or more, 0.04 wt% or more, 0.05 wt% or more, 0.06 wt% or more, 0.07 wt% or more, 0.08 wt% or more, 0.09 wt% or more, or 0.1 wt% or more with respect to the total weight of the topical preparation.

The upper limit of the concentration of the active ingredient may be 2.0 wt% or less, 1.5 wt% or less, 1.0 wt% or less, 0.9 wt% or less, 0.8 wt% or less, 0.7 wt% or less, 0.6 wt% or less, 0.5 wt% or less, 0.4 wt% or less, 0.3 wt% or less, 0.25 wt% or less, or 0.2 wt% or less with respect to the total weight of the topical preparation.

The concentration of the active ingredient is, for example, preferably from 0.01 wt% to 2.0 wt%, more preferably from 0.03 wt% to 1.0 wt%, still more preferably from 0.04 wt% to 0.8 wt%, even more preferably from 0.05 wt% to 0.4 wt% with respect to the total weight of the topical preparation.

The daily dose of sirolimus or the sirolimus derivative per unit surface area of a living body is not particularly limited.

For example, the lower limit of the daily dose may be 0.0001 mg/cm² or more, 0.0002 mg/cm² or more, 0.0005 mg/cm² or more, 0.001 mg/cm² or more, 0.002 mg/cm² or more, or 0.003 mg/cm² or more.

For example, the upper limit of the daily dose may be 2 mg/cm² or less, 1 mg/cm² or less, 0.5 mg/cm² or less, 0.05 mg/cm² or less, 0.04 mg/cm² or less, or 0.03 mg/cm² or less.

The dose is, for example, from 0.0001 mg/cm² to 2 mg/cm², preferably from 0.0002 mg/cm² to 1 mg/cm², more preferably from 0.0005 mg/cm² to 0.5 mg/cm², still more preferably from 0.001 mg/cm² to 0.05 mg/cm². It is appropriate that the above-mentioned amount of sirolimus or the sirolimus derivative be administered daily in a single dose or a plurality of divided doses by application or the like. In other words, the topical preparation of the present invention is preferably capable of achieving the above-mentioned dose.

The topical preparation may be prepared by a known method. For example, a gel may be prepared by allowing a solution containing sirolimus or the sirolimus derivative to gelate. An ointment may be prepared by mixing an ointment base with sirolimus or the sirolimus derivative. The gel, the ointment, and the cream are specifically described below.

### (A) Gel

When a solution containing sirolimus or the sirolimus derivative is allowed to gelate, it is appropriate that the solution be allowed to gelate using a gelling agent. Examples of the gelling agent include a carboxyvinyl polymer, carboxymethyl cellulose, aluminum hydroxide, and bentonite.

A specific configuration of the carboxyvinyl polymer is not particularly limited, and Carbopol (trademark), HIVISWAKO (trademark), AQUPEC (trademark), or the like may be used. Of those, Carbopol (trademark) 934P NF or Carbopol (trademark) 980 is preferred from the viewpoint of good feeling in the case of application as a topical preparation.

For example, when Carbopol (trademark) 934P NF or Carbopol (trademark) 980 is used, first, Carbopol (trademark) 934P NF or Carbopol (trademark) 980 is added to a solution containing sirolimus or the sirolimus derivative. Further, the pH of the solution is adjusted to be neutral by adding a pH adjuster (e.g., triethanolamine or tris(hydroxymethyl)aminomethane). Thus, the gelation of the solution may be induced.

The gel according to one embodiment of the present invention suitably contains an alcohol. The incorporation of the alcohol can allow the active ingredient contained in the gel to be efficiently absorbed into a skin tissue. The alcohol is preferably a lower alcohol, more preferably an alcohol having 2 to 4 carbon atoms. Still more preferred examples of the alcohol may include ethanol and isopropanol. From the viewpoint of allowing the active ingredient to be more efficiently and safely absorbed into a skin tissue, ethanol is particularly preferred.

The amount of the alcohol is not particularly limited, and only needs to be such an amount that sirolimus or the sirolimus derivative can be sufficiently dissolved. For example, the weight of the alcohol may be from 100 to 300 times as great as the weight of sirolimus or the sirolimus derivative, or may be from 120 to 250 times as great. With respect to the total weight of the topical preparation, the amount of the alcohol is preferably 20 wt% or more, more preferably 30 wt% or more, still more preferably 40 wt% or more. When the amount of the alcohol is 60 wt% or less, excessive evaporation of the alcohol from the preparation is prevented, and hence a preparation having a stable active ingredient concentration can be preserved (stored). Accordingly, the amount of the alcohol is more preferably about 50 wt% (from 45% to 55%).

When the alcohol is contained in such an amount that the active ingredient is sufficiently dissolved, the active ingredient can be allowed to be more efficiently absorbed into a skin tissue. As long as the amount of the alcohol is 50 wt% or less, as the amount of the alcohol increases, the active ingredient is more sufficiently dissolved, and hence can be allowed to be more efficiently absorbed into the skin tissue.

The amount of a gelling agent contained in the gel is not particularly limited, and only needs to be an amount sufficient for the solution containing sirolimus or the sirolimus derivative to gelate. The amount of the gelling agent contained in the gel may be, for example, 1.0 wt% or more with respect to the total weight of the gel. More specifically, the amount may be from 1.1 wt% to 20 wt%, from 1.2 wt% to 15 wt%, from 1.3 wt% to 10 wt%, from 1.4 wt% to 5 wt%, or from 1.5 wt% to 2.5 wt% with respect to the total weight of the gel.

The amount of the pH adjuster (neutralizer) contained in the gel is not particularly limited, and may be appropriately set depending on the amounts of the solvent and the gelling agent. The amount of the pH adjuster contained in the gel may be, for example, from 0.3 wt% to 5.0 wt%, from 0.4 wt% to 2.5 wt%, or from 0.5 wt% to 1.0 wt% with respect to the total weight of the gel.

For example, when the gelling agent (e.g., Carbopol (trademark) 934P NF or Carbopol (trademark) 980) and the pH adjuster (e.g., triethanolamine or tris(hydroxymethyl)aminomethane) are used as components for inducing gelation, the amount of the gelling agent may be, for example, 1.6 wt% with respect to the total weight of the topical preparation, and the amount of the pH adjuster may be, for example, 0.4 wt%, 0.6 wt%, or 0.8 wt% with respect to the total weight of the topical preparation. Of course, the present invention is not limited to the above-mentioned ratios.

The gel may contain a component other than the above-mentioned active ingredient, solvent (alcohol), gelling agent, and pH adjuster (neutralizer). Examples of the other component include a water-soluble polymer, water, and a medicinal component other than sirolimus or the sirolimus derivative.

Examples of the water-soluble polymer include polyethylene glycol, starch, methylcellulose, hydroxypropyl cellulose (HPC), polyvinyl alcohol, polyvinyl methyl ether, and polyvinylpyrrolidone.

The amount of the other component contained in the gel is not particularly limited, but may be, for example, 50 wt% or less, 40 wt% or less, 30 wt% or less, 20 wt% or less, or 10 wt% or less with respect to the total weight of the gel.

It is appropriate that the gel be applied daily or once every 2 to 3 days. The gel is preferably applied daily. When applied daily, the gel is preferably applied 1 to 3 times a day, more preferably applied 2 to 3 times a day.

In addition, a suitable active ingredient concentration in the preparation and a suitable frequency of the administration thereof are preferably adjusted depending on, for example, age, an administration site, and a skin thickness. For example, to a young child's thin skin, armpit, or the like, the gel having an active ingredient concentration of from 0.05 wt% to 0.2 wt% may be applied 1 to 3 times a day. In addition, to a palm, a sole, or the like, the gel having an active ingredient concentration of from 0.2 wt% to 0.8 wt% may be applied once or twice a day.

A more specific example of the composition of the gel is described below, but the present invention is not limited to the following composition. With the following configuration, a desired effect can be obtained with a smaller amount of sirolimus or the sirolimus derivative, and hence the adverse influence thereof on a living body can be alleviated.

The gel may contain a gelling agent (e.g., Carbopol (trademark) 934P NF or Carbopol (trademark) 980), water, an alcohol (e.g., ethanol or isopropanol, preferably ethanol), and a neutralizer (e.g., tris(hydroxymethyl)aminomethane or triethanolamine), in addition to sirolimus or the sirolimus derivative. In this case, a ratio among the weights of sirolimus or the sirolimus derivative, the gelling agent, water, the alcohol, and the neutralizer may be, for example, as follows: sirolimus or sirolimus derivative: gelling agent:water:alcohol:neutralizer=(0.5 to 2):16:490:(450 to 500):6. The above-mentioned ratio may also be (0.5 to 2):16:490:(480 to 490):6, or 2:16:490:486:6.

### (B) Ointment

As a base of the ointment, there are given, for example, waxes (e.g., natural waxes, such as white beeswax, lanolin, carnauba wax, and spermaceti; mineral waxes, such as montan wax; and synthetic waxes), paraffins (e.g., liquid paraffin and solid paraffin), and petrolatum (e.g., white petrolatum and yellow petrolatum).

The amount of the base is not particularly limited, but may be, for example, 10 wt% or more, 20 wt% or more, 30 wt% or more, 40 wt% or more, 50 wt% or more, 60 wt% or more, 70 wt% or more, 80 wt% or more, or 90 wt% or more with respect to the total weight of the ointment.

The ointment may contain a component other than sirolimus or the sirolimus derivative. Examples of the other component and the content thereof include the components and the contents thereof described above for the gel.

The ointment may contain propylene carbonate, solid paraffin, and white petrolatum in addition to sirolimus or the sirolimus derivative. In addition, the ointment may further contain liquid paraffin in addition to propylene carbonate, solid paraffin, and white petrolatum. In addition, the ointment may further contain white beeswax in addition to propylene carbonate, solid paraffin, white petrolatum, and liquid paraffin.

In this case, a ratio among the weights of sirolimus or the sirolimus derivative, propylene carbonate, solid paraffin, white petrolatum, liquid paraffin, and white beeswax may be as follows: sirolimus or sirolimus derivative: propylene carbonate: solid paraffin:white petrolatum:liquid paraffin:white beeswax=(0.3 to 10):(50 to 59.4):(30 to 45):(895):(0 to 10):(0 to 5). In the above-mentioned ratio, the total of the active ingredient, propylene carbonate, solid paraffin, and liquid paraffin is 105.

More specifically, the above-mentioned ratio may be the following ratio 1, ratio 2, or ratio 3.

(Ratio 1) Sirolimus or sirolimus derivative:propylene carbonate:solid paraffin:white petrolatum:liquid paraffin:white beeswax=2:58:30:895:10:5

(Ratio 2) Sirolimus or sirolimus derivative:propylene carbonate:solid paraffin:white petrolatum:liquid paraffin:white beeswax=2:58:45:895:0:0

(Ratio 3) Sirolimus or sirolimus derivative:propylene carbonate:solid paraffin:white petrolatum:liquid paraffin:white beeswax=2:58:35:895:10:0

When the above-mentioned ratio 1 and ratio 2 are compared to each other, the topical preparation produced at the ratio 1 has higher transparency than the topical preparation produced at the ratio 2, and can be reduced in amount of water present on the surface of the topical preparation.

When the above-mentioned ratio 1 and ratio 3 are compared to each other, the topical preparation produced at the ratio 1 is smoother than the topical preparation produced at the ratio 3, and can be reduced in amount of water present on the surface of the topical preparation.

It is appropriate that the ointment be applied daily or once every 2 to 3 days. The ointment is preferably applied daily. When applied daily, the ointment is preferably applied 1 to 3 times a day, more preferably applied 2 to 3 times a day.

In addition, a suitable active ingredient concentration in the preparation and a suitable frequency of the administration thereof are preferably adjusted depending on, for example, age, an administration site, and a skin thickness. For example, to a young child's thin skin, armpit, or the like, the ointment having an active ingredient concentration of from 0.05% to 0.2% may be applied 1 to 3 times a day. In addition, to a palm, a sole, or the like, the ointment having an active ingredient concentration of from 0.2% to 0.8% may be applied once or twice a day.

The ointment may be produced in accordance with a well-known method. An example of the production method is described below.

For example, the ointment may be prepared using a homomixer (for example, manufactured by Primix Corporation) or a universal mixer (for example, manufactured by Dalton Corporation). When the base is solid at room temperature, it is appropriate that the base be heated until becoming liquid, and the base in a liquid state be mixed with a solution having the active ingredient dissolved therein. More specifically, various components that are solid at room temperature (e.g., waxes, paraffins, and petrolatum) are heated to a temperature equal to or higher than their melting point (e.g., 70°C) to be dissolved, a solution having the active ingredient dissolved therein is added to the dissolved matter, and the whole is stirred. After that, while being stirred, the mixture is cooled to around room temperature (e.g., 40°C). Thus, the ointment may be produced.

Another method is as described below. The base is completely dissolved at from 70°C to 80°C, and stirred using a planetary centrifugal mixer (manufactured by Thinky Corporation), in a stirring mode, at 800 rpm for 30 minutes, then at 1,000 rpm for 5 minutes, and then at 2,000 rpm for 1 minute (15°C). After that, to the base, a solution having the active ingredient dissolved therein is added, and the whole is further stirred at 1,000 rpm for 1 minute and then at 2,000 rpm for 1 minute (without cooling). Thus, an ointment of better quality having dispersed therein extremely fine particles each containing the active ingredient may be prepared.

When the above-mentioned other component is to be incorporated into the ointment, it is appropriate that the ointment be prepared by: preparing a solution in which the active ingredient and the other component are dissolved in a desired solvent; adding the base to the solution; and performing the steps after the addition in accordance with the above-mentioned method.

### (C) Cream

The cream may be prepared by mixing a solution containing sirolimus or the sirolimus derivative and a base of the cream. Examples of the solution that dissolves sirolimus or the sirolimus derivative include an alcohol and an ethylene glycol ether. As the cream of the present invention, a cream that allows little of sirolimus or the sirolimus derivative to move into blood is preferred, and a cream containing an ethylene glycol ether is suitable. A diethylene glycol ether is preferably, for example, a diethylene glycol monoalkyl ether. An alkyl group of the diethylene glycol monoalkyl ether is preferably, for example, a C₁ to C₆ alkyl group. The diethylene glycol monoalkyl ether is preferably any of diethylene glycol monomethyl ether, diethylene glycol monoethyl ether, diethylene glycol monopropyl ether, and mixtures thereof, more preferably any of diethylene glycol monomethyl ether, diethylene glycol monoethyl ether, and mixtures thereof, most preferably diethylene glycol monoethyl ether (2-(2-ethoxyethoxy)ethanol, TRANSCUTOL (trademark) P).

Sirolimus or the sirolimus derivative is soluble in the diethylene glycol ether. Sirolimus dissolves well in the diethylene glycol monoethyl ether, and can be dissolved therein at up to 2.02 W/V%.

Sirolimus or the sirolimus derivative may be dissolved in the diethylene glycol ether before being formulated. The preparation using the diethylene glycol ether helps sirolimus or the sirolimus derivative to penetrate skin, and can retain the active ingredient in a skin tissue. A preparation using diethylene glycol monoethyl ether is particularly excellent in ability to help the active ingredient to penetrate the skin and in ability to retain the active ingredient in the skin tissue.

The content of the diethylene glycol ether in the cream only needs to be such an amount that sirolimus or the sirolimus derivative can be dissolved in the preparation. The content of the diethylene glycol ether is, for example, preferably from 1 wt% to 40 wt%, more preferably from 2 wt% to 30 wt%, still more preferably from 5 wt% to 25 wt%, even more preferably from 10% to 20% with respect to the total weight of the topical preparation.

The base of the cream contains an oily component, an aqueous component, and a surfactant. The base may further contain any other additive.

Examples of the oily component include hydrocarbons, fatty acid esters, waxes, higher fatty acids, and higher alcohols. Of those, a hydrocarbon and a higher alcohol are preferred.

Examples of the hydrocarbon include white petrolatum and liquid paraffin. Of those, white petrolatum is preferred. Examples of the higher alcohol include stearyl alcohol, cetanol, myristyl alcohol, lauryl alcohol, and oleyl alcohol. Of those, stearyl alcohol is preferred. An example of the fatty acid ester is isopropyl myristate. Examples of the wax include beeswax and lanolin. An example of the higher fatty acid is stearic acid.

The content of the oily component is, for example, preferably from 20% to 60%, more preferably from 30% to 50% with respect to the total weight of the topical preparation.

Examples of the aqueous component contained in the cream include water, a polyhydric alcohol, and a lower alcohol. Of those, water and a polyhydric alcohol are preferred.

Examples of the polyhydric alcohol include glycerin, propylene glycol, and 1,3-butylene glycol. Of those, propylene glycol is preferred. Examples of the lower alcohol include ethanol and isopropanol.

The content of the aqueous component including water in the cream is, for example, preferably from 20% to 60%, more preferably from 30% to 50% with respect to the total weight of the topical preparation.

The surfactant (emulsifier) is not particularly limited, and a surfactant (emulsifier) well known to a person skilled in the art may be used. Preferred examples of the surfactant (emulsifier) may include polyoxyethylene hydrogenated castor oil 60 and glyceryl monostearate. The content of the surfactant is, for example, preferably from 1% to 10%, more preferably from 2% to 8% with respect to the total weight of the topical preparation.

Examples of the other additive may include a preservative, an antioxidant, and a pH adjuster. In addition, the cream may also contain a medicinal component different from sirolimus or the sirolimus derivative.

The composition of the cream is preferably, for example, as follows with respect to the total weight of the topical preparation: 0.01 wt% to 2.0 wt% of sirolimus or the sirolimus derivative, 1 wt% to 40 wt% of the diethylene glycol ether, 5 wt% to 40 wt% of a hydrocarbon, 5 wt% to 30 wt% of a higher alcohol, 1 wt% to 30 wt% of a polyhydric alcohol, and 10 wt% to 50 wt% of water.

The composition of the cream is more preferably, for example, as follows with respect to the total weight of the topical preparation: 0.04 wt% to 0.8 wt% of sirolimus or the sirolimus derivative, 2 wt% to 30 wt% of the diethylene glycol ether, 10 wt% to 30 wt% of a hydrocarbon, 10 wt% to 25 wt% of a higher alcohol, 3 wt% to 20 wt% of a polyhydric alcohol, and 20 wt% to 40 wt% of water.

The cream is administered by, for example, being applied to an affected site daily or once every 2 to 3 days. The cream is preferably applied daily, more preferably applied 1 to 3 times a day, still more preferably applied 2 to 3 times a day.

In addition, the concentration of the active ingredient in the topical preparation and the frequency of the administration thereof are preferably adjusted depending on, for example, age, an administration site, and a skin thickness. For example, to a young child's thin skin, armpit, or the like, the topical preparation having an active ingredient concentration of from 0.05% to 0.2% may be applied 1 to 3 times a day. In addition, to a palm, a sole, or the like, the topical preparation having an active ingredient concentration of from 0.2% to 0.8% may be applied once or twice a day.

The cream may be produced in accordance with a well-known method. For example, a cream of good quality containing the active ingredient may be prepared by sequentially dissolving and mixing all raw materials for the base at from 70°C to 80°C, emulsifying the materials, then cooling the emulsified materials while mixing the materials, and mixing the cooled product with a solution of the active ingredient. The use of a planetary centrifugal mixer (manufactured by Thinky Corporation) for the emulsification and mixing provides good efficiency.

The present invention is more specifically described below by way of Examples, but the present invention is not limited thereto.

### Examples

[Production Examples: Preparation of Sirolimus-containing Gels]

Gels containing sirolimus at various concentrations were prepared. A specific method is as described below. Sirolimus was added to and dissolved in ethanol, and then water for injection was further added and mixed to prepare a mixed solution. Carbopol (trademark) 934P NF was added to and mixed with the mixed solution to prepare a homogeneous suspension. Tris(hydroxymethyl)aminomethane serving as a neutralizer was added to and mixed with the suspension to prepare each of the gels.

Components other than sirolimus in 1 g of each of the gels were 16 mg of Carbopol (trademark) 934P NF, 490 mg of water, 480 mg to 490 mg of ethanol, and 6 mg of tris(hydroxymethyl)aminomethane.

More specifically, the gels were prepared according to the compositions of the following Production Examples 1 to 5.

Production Example 1: A gel containing 0.4 wt% of sirolimus (total amount: 100 g)
Sirolimus 0.4 g
Ethanol 48.4 g
Water for injection 49 g
Carbopol (trademark) 934P NF 1.6 g
Tris(hydroxymethyl)aminomethane 0.6 g

Production Example 2: A gel containing 0.8 wt% of sirolimus (total amount: 100 g)
Sirolimus 0.8 g
Ethanol 48 g
Water for injection 49 g
Carbopol (trademark) 934P NF 1.6 g
Tris(hydroxymethyl)aminomethane 0.6 g

Production Example 3: A gel containing 0.2 wt% of sirolimus (total amount: 100 g)
Sirolimus 0.2 g
Ethanol 48.6 g
Water for injection 49 g
Carbopol (trademark) 934P NF 1.6 g
Tris(hydroxymethyl)aminomethane 0.6 g

Production Examples 4 and 5: A gel containing 0.05% of sirolimus (Production Example 4) and a gel containing 0.1% of sirolimus (Production Example 5) were produced in the same manner as in Production Examples 1 to 3.

### [Production Example: Preparation of Sirolimus-containing Cream]

Production Example 6: A cream containing 0.2 wt% of sirolimus (total amount: 100 g) Sirolimus powder was obtained from Fujian Kerui Pharmaceutical Co., Ltd. The sirolimus powder was added to and dissolved in diethylene glycol monoethyl ether (TRANSCUTOL (trademark) P), and then the solution was mixed with a preliminarily mixed and prepared base (oil-in-water (O/W) emulsion) to yield a cream. Specific composition is shown below.
Sirolimus 0.2 g
Diethylene glycol monoethyl ether 10 g
Hydrophilic cream 89.8 g
White petrolatum 22.45 g
Stearyl alcohol 17.96 g
Propylene glycol 10.78 g
Polyoxyethylene hydrogenated castor oil 60 3.59 g
Glyceryl monostearate 0.90 g
Methyl p-benzoate 0.09 g
Propyl p-benzoate 0.09 g
Purified water 33.94 g

### [Cases]

### Case 1

### A 9-year-old female (having tuberous sclerosis complex and intractable epilepsy)

Epileptic seizures had been occurring at a frequency of from 8 times/month to 10 times/month. Twice a day topical application of the 0.2 wt% sirolimus gel prepared in Production Example 3 to her facial angiofibroma was started. As a result, epileptic seizures were reduced from the start of the topical application, and seizures occurred only about 3 times during a topical application period of 3 months. After that, the topical application was stopped, and as a result, seizures nearly returned to the original frequency. Twice a day topical application of Rapalimus Gel (trademark) 0.2% (gel containing 0.2 wt% sirolimus) was started again, and as a result, not a single seizure occurred in 2 months from the start of the topical application. After that, the topical application was stopped, and as a result, seizures nearly returned to the original frequency. Six months later, topical application of Rapalimus (trademark) Gel 0.2% was started again, and as a result, seizures stabilized to occur about 4 times a month. The blood concentration of sirolimus was measured (AP13200 LC-MS/MS System, Sciex, measurement limit: 1.0 ng/ml) at the time of the initial topical application, and as a result, was found to be below the measurement limit value. The topical application amount of the sirolimus gel was about 10 g in 2 months, and the daily usage amount of the sirolimus gel was about 0.17 g (0.3 mg in terms of sirolimus). Rapalimus Gel (trademark) 0.2% is a sirolimus-containing gel developed by the inventor of the present invention in collaboration with Nobelpharma Co., Ltd., and the gel contains 0.2% of sirolimus and contains a carboxyvinyl polymer, ethanol, and triethanolamine as additives.

### Case 2

### A 13-year-old male (having tuberous sclerosis complex, autism, developmental disorder, and intractable epilepsy)

Twice a day topical application of Rapalimus (trademark) Gel 0.2% to the face at a single dose of up to 0.1 g was started, and as a result, the frequency of epileptic seizures, which had been occurring nearly every day, was reduced by about a half. In addition, his perseverative tendency was ameliorated.

### Case 3

### A 28-year-old male (having tuberous sclerosis complex and developmental disorder)

His developmental disorder was evident (with autistic tendency), but twice a day topical application of Rapalimus (trademark) Gel 0.2% to the face at a single dose of up to 0.15 g was started, and as a result, after the start of the topical application, he became calmer and more benign, and showed a reduction in aggression toward others. In addition, his talking to himself was also reduced.

### Case 4

### A 24-year-old female (tuberous sclerosis complex, developmental disorder, epilepsy, and schizophrenia)

Twice a day topical application of Rapalimus (trademark) Gel 0.2% to the face at a single dose of about 0.15 g resulted in a reduction in number of epileptic seizures. In addition, her auditory and visual hallucinations, which had persisted despite past use of various internal medicines, disappeared. She became less irritable, became gentler and more benign, and became more cheerful and easier to contact. Further, she had been seclusive before, but started going out alone.

### Case 5

### A 11-year-old male (tuberous sclerosis complex and developmental disorder)

He had had a remarkable developmental disorder and strong symptoms of autism, but after the start of topical application, his perseverative tendency was ameliorated and his repetitive behavior was also ameliorated.

### [Pharmacological Tests in Model Mice]

### Test Example 1: Number of Epileptic Seizures

### Model Mice and Control Mice

Tuberous sclerosis complex model mice (C57BL/6J,Tsc2^{flox/flox}Mitf::Cre) are model mice generated by the inventor of the present invention and others, and are conditional knockout mice expressing Cre recombinase in a manner dependent on a melanocyte-specific Mitf promoter and having a deletion of exons 3-5 of Tsc2 (J Invest Dermatol. 2018; 138(3): 669-78). The model mice are tuberous sclerosis complex model mice exhibiting epilepsy, a behavioral abnormality, and vitiligo, and can also be used as epilepsy model mice (PLoS One. 2020; 15(1)). As a control, C57BL/6J,Tsc2^{+/flox}Mitf::Cre mice were used. The control mice do not exhibit an abnormality such as epilepsy and cannot be distinguished from C57BL/6J, and hence are hereinafter referred to as "normal mice".

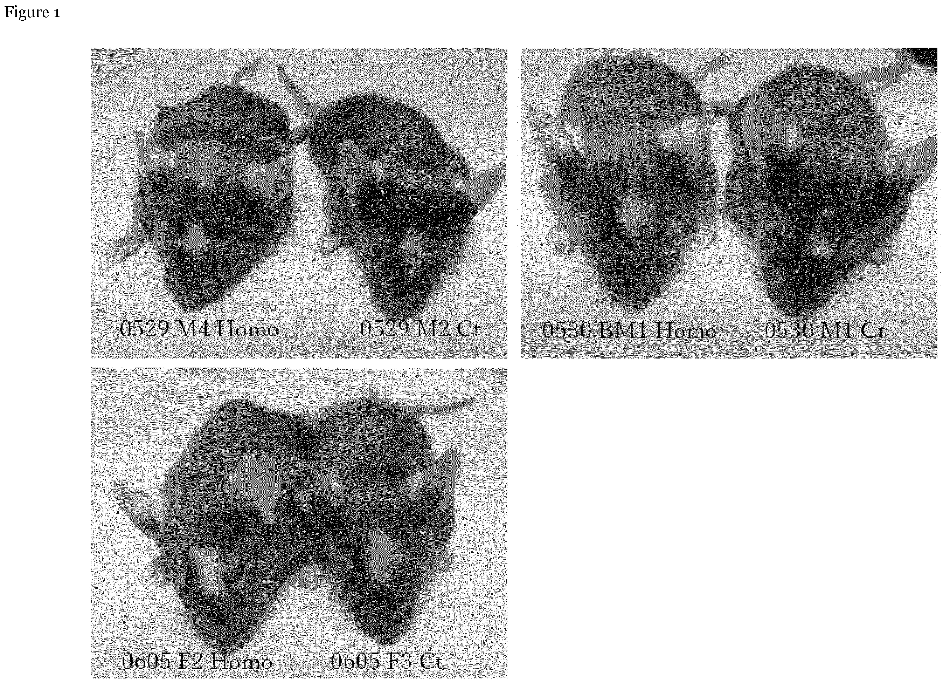

The central part of the forehead of the mice of both groups was shaved under anesthesia. The 0.2 wt% sirolimus gel prepared in Production Example 3 or a placebo (only the base) was applied to the shaved forehead once a day at 5 mg each (10 µg in terms of sirolimus) (FIG. 1). For the mice having the sirolimus gel or the placebo applied thereto, the number of epileptic seizures was counted for 7 days. The number of epileptic seizures was investigated by 3 times per day of handling. The handling is a treatment involving transferring a mouse to another empty cage and, for example, retaining the mouse in the same manner as in intraperitoneal injection or holding the mouse upside down by the tail for 2 minutes, and a case in which a seizure occurred was counted as one seizure. A case in which a seizure occurred before the treatment was also counted as one seizure. The results are shown in Table 1. The application of the sirolimus gel reduced the number of seizures.

**Table 1**

| | | Day 1 | Day 2 | Day 3 | Day 4 | Day 5 | Day 6 | Day 7 | Total |
|---|---|---|---|---|---|---|---|---|---|
| Normal mice (male) with placebo applied | 0529 M2 Ct | 0 | 0 | 0 | 0 | 0 | 0 | 0 | 0 |
| Tuberous sclerosis complex model mice (male) with placebo applied | 0529 M4 Hom o | 1 | 2 | 3 | 2 | 3 | 2 | 0 | 13 |
| Normal mice (male) with sirolimus gel applied | 0530 M1 Ct | 0 | 0 | 0 | 0 | 0 | 0 | 0 | 0 |
| Tuberous sclerosis complex model mice (male) with sirolimus gel applied | 0530 BM1 Hom o | 3 | 1 | 0 | 0 | 0 | 0 | 1 | 5 |
| Tuberous sclerosis complex model mice (female) with sirolimus gel applied | 0605 F2 Hom o | 1 | 0 | 0 | 0 | 0 | 1 | 0 | 2 |
| Normal mice (female) with sirolimus gel applied | 0605 F3 Ct | 0 | 0 | 0 | 0 | 0 | 0 | 0 | 0 |

### Test Example 2: Open Field Test

Mice having a sirolimus gel or a placebo applied thereto in the same manner as in Test Example 1 were subjected to an open field test on the 8th day (Day 8) from the start of the application. The mice were placed in a circular open field (diameter: 50 cm), and time periods spent in a central zone and any other zone, moving speed, and travel distance were measured for 15 minutes. The central zone was defined as a zone having a diameter of 30 cm in the center. The results are shown in Table 2. Border/Center is a ratio between the cumulative time period spent outside the central zone and the cumulative time period spent in the central zone, and indicates the ratio of the time period spent outside the central zone (edge zone of the field: Border), and a higher value therefor indicates a stronger anxiety tendency. The results of the test showed that the application of the sirolimus gel weakened a sense of anxiety, and also reduced the moving speed and the travel distance.

**Table 2**

| | | Border/Cen ter | Moving speed (cm/s) | Travel distance (cm) |
|---|---|---|---|---|
| Normal mice (male) with placebo applied | 0529 M2 Ct | 1.6 | 5.2 | 4,635.6 |
| Tuberous sclerosis complex model mice (male) with placebo applied | 0529 M4 Homo | 8.2 | 11.9 | 10,705.7 |
| Normal mice (male) 0530 M1 Ct with sirolimus gel applied | 0530 M1 Ct | 1.6 | 5.8 | 5,176.7 |
| Tuberous sclerosis complex model mice (male) with sirolimus gel applied | 0530 BM1 Homo | 6.3 | 9.3 | 8,333.1 |

### Test Example 3: Blood Concentration of Sirolimus

125 mg of the 0.05% sirolimus gel prepared in Production Example 4, the 0.2% sirolimus gel prepared in Production Example 3, or the 0.4% sirolimus gel prepared in Production Example 1 was applied to an 18 cm² (6 cm×3 cm) area on the back of hairless mice (HR mice, 8 weeks old, male), and 2 hours later, blood was collected for the measurement of a blood concentration (AP13200 LC-MS/MS System, Sciex, measurement limit: 0.1 ng/ml). The blood concentration of each of the mice is shown in Table 3. Despite the application of a large amount of sirolimus gel, the blood concentration was hardly increased.

**Table 3**

| | | Blood concentration (ng/ml) |
|---|---|---|
| Placebo applied | Mouse 1 | 0 |
| | Mouse 2 | 0 |
| | Mouse 3 | 0 |
| 0.05% Sirolimus gel applied | Mouse 1 | 0 |
| | Mouse 2 | 0 |
| | Mouse 3 | 0 |
| 0.2% Sirolimus gel applied | Mouse 1 | 0.12 |
| | Mouse 2 | 0.19 |
| | Mouse 3 | 0 |
| 0.4% Sirolimus gel applied | Mouse 1 | 0 |
| | Mouse 2 | 0 |
| | Mouse 3 | 0 |

### Test Example 4: Number of Epileptic Seizures (Topical Application)

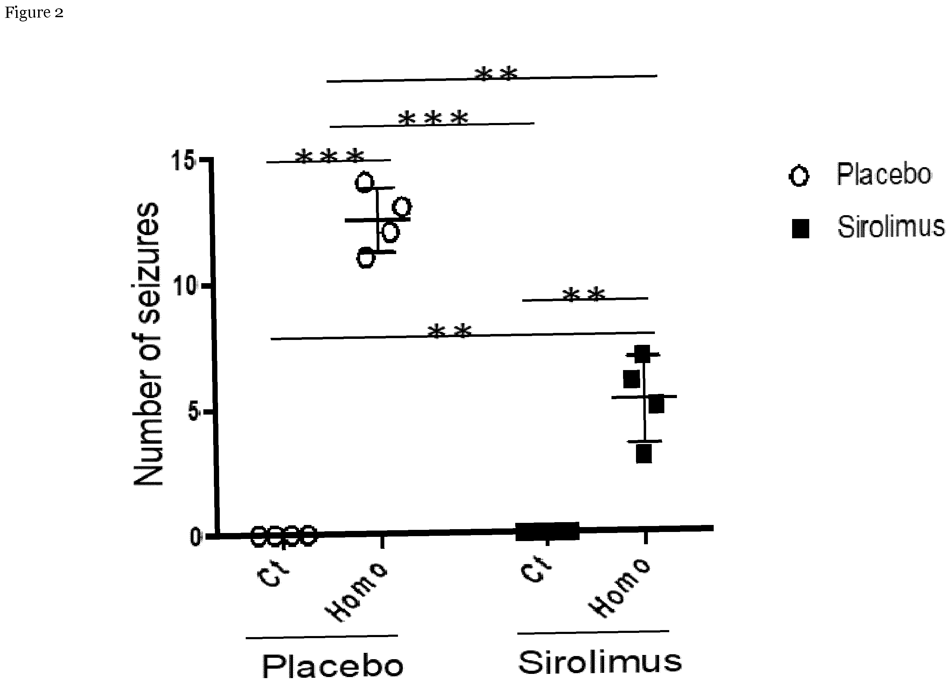

The 0.2 wt% sirolimus gel or a placebo (only the base) was applied to model mice and normal mice once a day at 5 mg each (10 µg in terms of sirolimus) in the same manner as in Test Example 1. For the mice having the sirolimus gel or the placebo applied thereto, the number of epileptic seizures was counted by the same method as in Test Example 1 for 7 days (n=4 in each group). The number of seizures over 7 days is shown in FIG. 2. The application of sirolimus reduced the number of seizures.

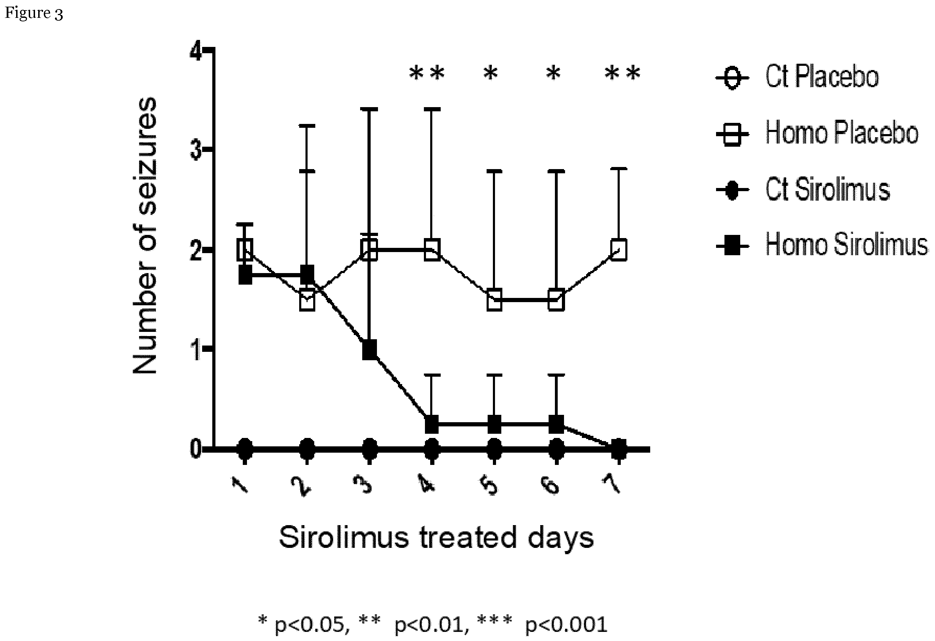

Test Example 5: Number of Epileptic Seizures (Intraperitoneal Administration) Sirolimus (3 mg/kg/day) or a placebo (saline) was intraperitoneally administered to model mice and normal mice similar to those in Test Example 1 once a day for 7 days. The number of seizures was measured by observing the mice for 2 minutes 5 times per day (n=6 in each group). The results are shown in FIG. 3. The intraperitoneal administration of sirolimus reduced the number of seizures. The number of seizures over 7 days in the group intraperitoneally administered sirolimus was about 5, which was nearly equal to the number of seizures in the case where the 0.2 wt% sirolimus gel was administered to the forehead once a day at 5 mg (10 µg in terms of sirolimus).

Test Example 6: Blood Concentration after 7-Day Continuous Administration Sirolimus (3 mg/kg/day) or a placebo (saline) was intraperitoneally administered to mice (C57BL/6J, body weight: 20 g) once a day for 7 days. After 24 hours from the administration on the 7th day, blood was collected from each of the mice, and blood sirolimus concentration was measured. The results are shown in Table 4.

**Table 4**

| | | |
|---|---|---|
| Sirolimus | Placebo | (ng/ml·blood) |
| 94.43 | N.D. | |
| 85.59 | N.D. | |
| 48.55 | - | |
| 216.83 | - | |

ICR mice (body weight: about 37 g) were obtained (Japan SLC, Inc.). The 0.2 wt% sirolimus gel prepared in Production Example 3 or a placebo (only the base) was applied to their shaved forehead once a day at 5 mg each (10 µg in terms of sirolimus). After 24 hours from the administration on the 7th day, blood was collected from each of the mice, and blood sirolimus concentration was measured. The results are shown in Table 5.

**Table 5**

| | | |
|---|---|---|
| Sirolimus | Placebo | (ng/ml·blood) |
| 3.87 | N.D. | |
| 2.85 | N.D. | |
| 5.98 | N.D. | |

The measurement of the blood concentration was performed by the same method as in Test Example 3. The blood concentration after the topical application to the skin was one 26th in terms of average value as compared to the blood concentration after systemic administration by the intraperitoneal administration. It was revealed that, although the suppressing effect on seizures was nearly the same, the topical application reduced the blood concentration to lower levels. The action mechanism by which epileptic seizures are suppressed through systemic administration and the mechanism by which epileptic seizures are suppressed through topical application may differ from each other.

### Test Example 7: 3-Week Continuous Administration

6-week-old to 8-week-old model mice and normal mice similar to those in Test Example 1 were used, and the 0.2 wt% sirolimus gel prepared in Production Example 3 and a placebo (only the base) were applied thereto for 3 weeks by the same method as in Test Example 1. The average number of seizures per day in each week during the administration period is shown in Table 6. A method of measuring the number of seizures was performed in the same manner as in Test Example 1. In the 0.2 wt% sirolimus gel treatment group, the number of epileptic seizures reduced from around the 4th day of the 1st week, and the average number of seizures over a week was 1 or less (0.143 per day) in each of the 2nd week and the 3rd week.

**Table 6**

| | Placebo applied | | 0.2% Sirolimus gel applied | |
|---|---|---|---|---|
| | Normal mice | Model mice | Normal mice | Model mice |
| 1 st week | 0 | 1.571 | 0 | 1.000 |
| 2nd week | 0 | 1.429 | 0 | 0.143 |
| 3rd week | 0 | 1.714 | 0 | 0.143 |
| Total number of seizures over 21 days | 0 | 33 | 0 | 9 |

### Test Example 8: Recurrence of Seizures by Stopping Topical Application

Model mice (7 weeks old, body weight: 21.55 g) and normal mice similar to those in Test Example 1 were used, and the 0.2 wt% sirolimus gel was applied thereto for 4 days (Day 1 to Day 4) by the same method as in Test Example 1. The application was stopped from the 5th day (Day 5), and the recurrence of seizures was observed. A method of measuring seizures was performed in the same manner as in Test Example 1. On the 18th day (Day 18), recurrence occurred, and one seizure was observed. Seizures were observed every day since Day 18. The numbers of seizures on Day 1 and from Day 4 to Day 22 are shown in Table 6.

**Table 7**

| | Day 1 | Day 4 | Day 5 | Day 6 | Day 7 | Day 8 | Day 9 | Day 10 | Day 11 | Day 12 |
|---|---|---|---|---|---|---|---|---|---|---|
| Model mice | 1 | 2 | 0 | 0 | 0 | 0 | 0 | 0 | 0 | 0 |
| Normal mice | 0 | 0 | 0 | 0 | 0 | 0 | 0 | 0 | 0 | 0 |
| | | | | | | | | | | |

| Day | Day 13 | Day 14 | Day 15 | Day 16 | Day 17 | Day 18 | Day 19 | Day 20 | Day 21 | Day 22 |
|---|---|---|---|---|---|---|---|---|---|---|
| Model mice | 0 | 0 | 0 | 0 | 0 | 1 | 1 | 2 | 2 | 3 |
| Normal mice | 0 | 0 | 0 | 0 | 0 | 0 | 0 | 0 | 0 | 0 |

The results mentioned above have revealed that the topical preparation of the present invention exhibits therapeutic effects on epilepsy, an autism spectrum disorder, and an anxiety disorder while causing little increase in blood concentration.

### Industrial Applicability

The topical preparation of the present invention greatly contributes to future medicine.

## Claims

1. A topical preparation for treating epilepsy, comprising as an active ingredient at least one selected from the group consisting of sirolimus and a sirolimus derivative.

2. The topical preparation according to claim 1, wherein the sirolimus derivative is at least one selected from the group consisting of everolimus, temsirolimus, ridaforolimus, and zotarolimus.

3. The topical preparation according to claim 1 or 2, wherein the epilepsy is epilepsy accompanying tuberous sclerosis complex.

4. The topical preparation according to any one of claims 1 to 3, wherein the topical preparation is for use by administration to a head.

5. The topical preparation according to any one of claims 1 to 4, further comprising a lower alcohol.

6. A topical preparation for treating an autism spectrum disorder, comprising as an active ingredient at least one selected from the group consisting of sirolimus and a sirolimus derivative.

7. The topical preparation according to claim 6, wherein the autism spectrum disorder is autism.

8. The topical preparation according to claim 6, wherein the autism spectrum disorder is a disorder in building an interpersonal relationship in an autism spectrum disorder, and/or perseveration and obsession in an autism spectrum disorder.

9. The topical preparation according to claim 6, wherein the autism spectrum disorder is an autism spectrum disorder accompanying tuberous sclerosis complex.

10. A topical preparation for treating an anxiety disorder, comprising as an active ingredient at least one selected from the group consisting of sirolimus and a sirolimus derivative.

11. The topical preparation according to claim 10, wherein the anxiety disorder is generalized anxiety disorder.

12. The topical preparation according to claim 10, wherein the anxiety disorder is an anxiety disorder accompanying tuberous sclerosis complex.
